# DEMANDE DE BREVET EUROPEEN

(11) **EP 2 377 567 A1**
(43) Date de publication de la demande: **19.10.2011**
(21) Numéro de dépôt: 11161276.8
(22) Date de dépôt: 06.04.2011
(51) Int. Cl.: A61M 16/04, A61M 16/06

(54) **Appareil d'assistance respiratoire**

(30) Priorité: 13.04.2010 FR 1001555
(71) Demandeur: Boussignac, Georges, 92160 Antony (FR)
(72) Inventeur: Boussignac, Georges, 92160 Antony (FR)
(74) Mandataire: Bloch & Bonnétat

(57) **Abrégé**

- Selon l'invention, l'appareil d'assistance respiratoire comportant un corps creux (2) délimitant une chambre interne (3), pourvue d'une entrée et d'une sortie de gaz respiratoire, et un organe d'appui (11), destiné à prendre appui sur une partie (9) d'un patient et enveloppé par une membrane souple (13), est remarquable en ce que :
■ la membrane (13) est rapportée sur la surface extérieure (2E) du corps creux (2) et est apte à s'interposer entre l'organe d'appui (11) et la partie (9) du patient sur laquelle il prend appui ; et
■ le corps creux (2) comporte des orifices de communication (14) ménagés en regard de la membrane (13) pour permettre le passage de gaz respiratoire depuis la chambre (3) vers un espace intermédiaire (E) formé entre la membrane (13) et la surface extérieure (2E) du corps (2).

## Description

La présente invention a pour objet un appareil d'assistance respiratoire, utilisable sur des patients dont la respiration spontanée est absente ou insuffisante.

Par le document EP1121953, on connaît déjà des appareils d'assistance respiratoire permettant d'amener, dans les poumons d'un patient, du gaz respiratoire provenant d'une source extérieure, lesdits appareils respiratoires comportant :
- un corps creux, dont le volume interne définit une chambre pourvue d'une entrée de gaz respiratoire destinée à être reliée à ladite source et d'une sortie de gaz respiratoire destinée à être reliée à une voie respiratoire dudit patient ;
- une ouverture qui constitue ladite sortie de gaz respiratoire ;
- un organe d'appui qui borde le voisinage du contour de ladite ouverture et qui est destiné à prendre appui sur une partie dudit patient pour assurer l'étanchéité entre ladite sortie de gaz respiratoire et l'extérieur ; et
- une membrane souple qui enveloppe intégralement ledit organe d'appui.

De tels appareils d'assistance respiratoire connus peuvent, par exemple, prendre la forme :
- d'un masque respiratoire, dans lequel ledit corps est une coque creuse destinée à être appliquée, par ladite ouverture qui constitue ladite sortie de gaz, sur le visage dudit patient en enfermant le nez de celui-ci. L'entrée de gaz respiratoire est alors formée par un embout solidaire du fond de ladite coque, tandis que ledit organe d'appui présente la forme d'un bourrelet interposé entre ladite ouverture et ledit visage du patient ; ou bien encore
- d'un dispositif d'intubation nasal, dans lequel ledit corps creux est un élément tubulaire destiné à être introduit dans une narine du patient. Dans ce cas, lesdites entrée et sortie de gaz respiratoire sont formées par les extrémités opposées dudit élément tubulaire et ledit organe d'appui est une vessie gonflable se présentant sous la forme d'un ballonnet porté par la surface extérieure dudit élément et interposé entre celui-ci et la paroi interne de ladite narine.

La présente invention a pour objet de perfectionner les appareils d'assistance respiratoire précités et, notamment, de parfaire l'étanchéité entre l'organe d'appui et la partie du patient en contact (visage ou paroi interne de la narine par exemple) sur laquelle il est appliqué.

A cette fin, selon l'invention, l'appareil d'assistance respiratoire permettant d'amener, dans les poumons d'un patient, du gaz respiratoire provenant d'une source extérieure, ledit appareil comportant :
- un corps creux, dont le volume interne définit une chambre interne pourvue d'une entrée de gaz respiratoire destinée à être reliée à ladite source et d'une sortie de gaz respiratoire destinée à être reliée à une voie respiratoire dudit patient ;
- une ouverture qui constitue ladite sortie de gaz respiratoire ;
- un organe d'appui qui borde le voisinage du contour de ladite ouverture et qui est destiné à prendre appui sur une partie dudit patient pour assurer l'étanchéité entre ladite sortie de gaz respiratoire et l'extérieur ; et
- une membrane souple qui enveloppe, au moins partiellement, ledit organe d'appui,
   est remarquable :
- en ce que ladite membrane souple est rapportée, à une de ses extrémités, sur la surface dudit corps creux orientée vers l'extérieur ;
- en ce que ladite membrane souple est apte à s'interposer entre ledit organe d'appui et la partie dudit patient sur laquelle il prend appui ; et
- en ce que ledit corps creux comporte au moins un orifice de communication ménagé en regard de ladite membrane,
de sorte que du gaz respiratoire est apte à traverser ledit orifice de communication, à circuler, depuis ladite chambre, dans un espace intermédiaire formé entre ladite membrane et la surface extérieure dudit corps creux, et à contourner l'organe d'appui pour retourner dans ladite chambre interne à proximité de ladite ouverture.

Ainsi, grâce à l'invention, du gaz respiratoire peut traverser le ou les orifices de communication du corps creux pour circuler dans l'espace intermédiaire, contourner l'organe d'appui et, finalement, retourner dans la chambre interne dans un voisinage de l'ouverture. Une telle circulation de gaz respiratoire conduit à la formation d'un coussin de gaz entre au moins une portion de l'organe d'appui et la membrane souple en regard, interposée entre ladite portion et la partie du patient (visage, nez) et tendue par la circulation de gaz. Ce coussin de gaz, intrinsèquement mou, s'adapte aisément à la morphologie du patient, par simple contact de celui-ci sur la partie concernée du patient, ce qui assure une étanchéité uniforme entre la partie du patient et l'appareil d'assistance respiratoire, suivant l'ouverture de la chambre interne.

En outre, grâce à l'invention, on peut réduire sensiblement la pression d'application de l'appareil respiratoire sur la partie d'appui du patient pour obtenir l'étanchéité recherchée, notamment aux endroits où cette étanchéité ne peut être obtenue par l'organe d'appui par un simple contact. On supprime ainsi le risque d'apparition d'escarre lors d'un port prolongé du masque, tout en atténuant l'inconfort d'utilisation pour le patient.

De surcroît, du fait que le gaz respiratoire, après avoir traversé le corps creux depuis la chambre interne, aboutisse de nouveau dans celle-ci, aucune perte de gaz respiratoire n'est constatée, ce qui permet de réduire la consommation de gaz respiratoire au cours de l'utilisation de l'appareil respiratoire.

De préférence, ledit corps creux comporte une pluralité d'orifices de communication régulièrement répartis sur ce dernier, de manière à assurer une répartition uniforme du gaz respiratoire dans l'espace intermédiaire et ainsi obtenir un coussin de gaz homogène le long de l'ouverture de la chambre interne.

De façon avantageuse, le ou les orifices de communication peuvent se présenter sous la forme d'un trou percé de façon oblique dans la paroi dudit corps creux, de sorte que l'axe des orifices soit incliné par rapport à la direction locale orthogonale à ladite paroi. Ainsi, on facilite le passage de gaz respiratoire dans l'espace intermédiaire, tout en réduisant les turbulences au voisinage du ou des orifices de communication dans la chambre interne.

De préférence, ladite membrane souple est réalisée dans un film plastique (par exemple du silicone) de quelques micromètres d'épaisseur.

Dans une forme de réalisation conforme à la présente invention, l'appareil respiratoire se présente sous la forme d'un masque nasal ou bucco-nasal, dans lequel ledit corps est une coque creuse destinée à être appliquée, par ladite ouverture, sur le visage d'un patient en enfermant au moins le nez de celui-ci, l'entrée du gaz respiratoire étant prévue dans le fond de ladite coque et ledit organe d'appui présentant la forme d'un bourrelet suivant le contour de l'ouverture de ladite coque et interposé entre ladite ouverture et le visage dudit patient.

Ainsi, lorsque le masque est appliqué sur le visage du patient, le coussin de gaz se déforme pour s'adapter et se conformer, avec ajustement, à la morphologie du visage du patient et, en particulier, à ses irrégularités, ce qui permet de corriger les défauts d'étanchéité du bourrelet sans recours à une pression d'application importante du masque sur le visage. En outre, on peut aisément adapter un masque respiratoire de dimensions données (de préférence destiné à des visages larges) sur tout type de visage, quelles que soient les dimensions de celui-ci.

De préférence, l'autre extrémité de ladite membrane est libre et se prolonge vers l'intérieur de ladite ouverture. Ainsi, la circulation de gaz respiratoire entre la membrane et le bourrelet permet de plaquer l'extrémité libre contre le visage, ce qui accroît la portion de membrane en contact avec le visage et, de fait, améliore l'étanchéité.

Dans une variante de la forme de réalisation précitée conforme à l'invention, l'autre extrémité de ladite membrane peut être rapportée sur la surface de ladite coque creuse tournée vers ladite chambre, entre ledit bourrelet et ledit orifice de communication, et au moins un orifice d'échappement peut être ménagé dans la portion de ladite membrane agencée à l'intérieur de ladite chambre, de manière à permettre une évacuation du gaz respiratoire dans cette dernière.

En outre, afin d'assurer un gonflage permanent de la membrane souple, il est préférable que la section dudit orifice de communication soit supérieure à celle dudit orifice d'échappement.

Dans une autre forme de réalisation de l'invention, l'appareil respiratoire se présente sous la forme d'un dispositif d'intubation nasal, dans lequel ledit corps creux est un élément tubulaire destiné à être introduit dans une narine du patient, lesdites entrée et sortie de gaz respiratoire étant formées par les extrémités opposées dudit élément tubulaire et ledit organe d'appui se présentant sous la forme d'un ballonnet porté par la surface extérieure dudit élément tubulaire et interposé entre celui-ci et la paroi interne de ladite narine.

De préférence, ladite membrane recouvre intégralement ledit organe d'appui et se prolonge, par son autre extrémité libre, vers l'intérieur de ladite ouverture.

Par ailleurs, quelle que soit la forme de réalisation de l'appareil d'assistance respiratoire conforme à l'invention, la membrane souple peut être rapportée de façon amovible sur ledit corps creux.

Les figures du dessin annexé feront bien comprendre comment l'invention peut être réalisée. Sur ces figures, des références identiques désignent des éléments semblables.
La figure 1 représente schématiquement, selon une coupe axiale, un exemple de réalisation de l'appareil d'assistance respiratoire conforme à la présente invention, se présentant sous la forme d'un masque.
Les figures 2 et 3 illustrent schématiquement, en vues partielles agrandies, le processus selon lequel l'étanchéité est obtenue avec le masque de la figure 1.
La figure 4, semblable à la figure 1, illustre un autre exemple de réalisation d'un masque respiratoire conforme à la présente invention.
La figure 5 représente schématiquement, en coupe axiale, une variante de réalisation de l'appareil d'assistance respiratoire conforme à la présente invention, se présentant sous la forme d'un élément tubulaire.

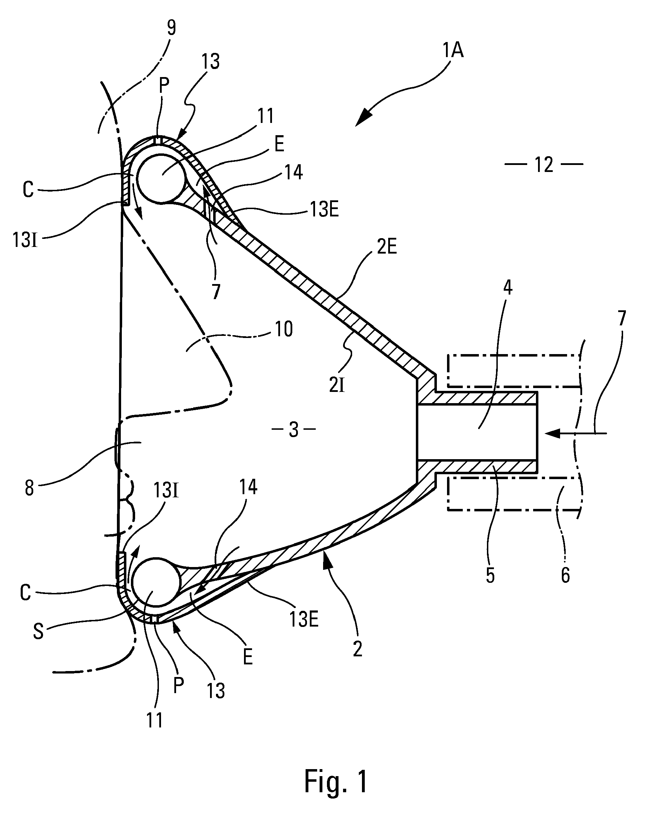

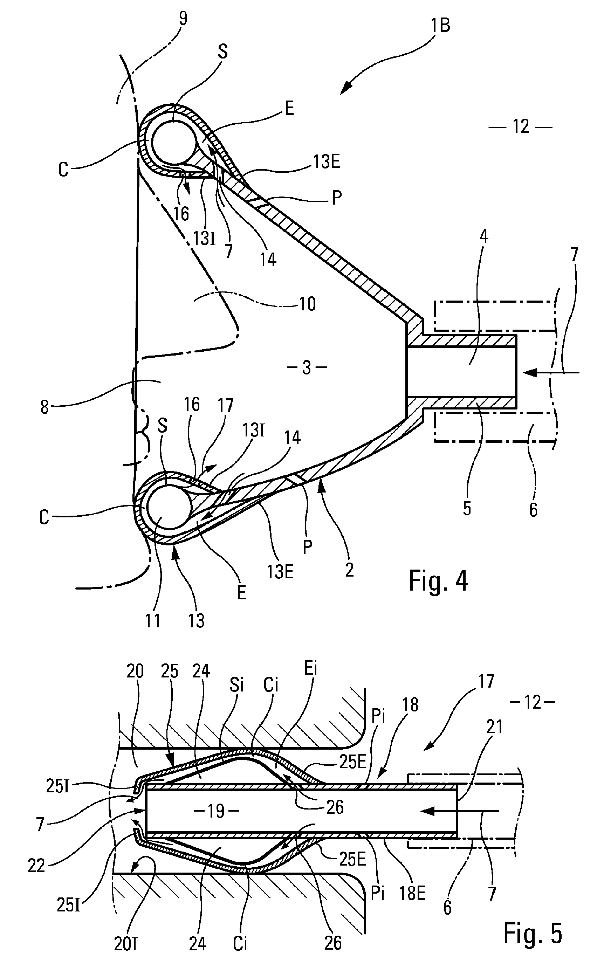

L'appareil d'assistance respiratoire 1A, 1 B, conforme à la présente invention et représenté sur les figures 1 et 4, se présente sous la forme d'un masque respiratoire comportant une coque rigide creuse 2 délimitant une chambre interne 3. Au fond de la coque 2 est aménagée une entrée 4 de gaz respiratoire, par exemple grâce à un embout tubulaire 5, solidaire de ladite coque 2, pouvant être reliée à une source de gaz respiratoire (non représentée), par exemple une bouteille sous pression, par une tubulure appropriée 6. Sur les figures 1 et 4, l'arrivée de gaz respiratoire frais est symbolisée par la flèche 7.

La chambre interne 3 comporte une sortie du gaz respiratoire constituée par l'ouverture 8 de ladite coque rigide 2. Celle-ci est destinée à être appliquée, par son ouverture 8, sur le visage 9 d'un patient (représenté en traits mixtes) en enfermant le nez 10 de celui-ci.

Afin d'assurer l'étanchéité au gaz entre l'ouverture 8 de la coque 2 et le visage 9, le masque respiratoire 1 A, 1 B comporte un organe d'appui sous la forme d'un bourrelet 11, solidaire de la coque 2 et suivant le contour de l'ouverture 8 de cette dernière. Le bourrelet 11 est interposé entre ladite ouverture 8 et le visage 9 du patient, lorsque la coque 2 est appliquée contre le visage 9, pour isoler la chambre interne 3 de l'extérieur 12.

De plus, le bourrelet 11 bordant l'ouverture 8 est formé dans une mousse souple, de préférence à cellules fermées. En variante, il pourrait se présenter sous la forme d'une vessie gonflable à paroi mince.

Le masque respiratoire 1A, 1 B comporte également une membrane souple 13, sous la forme d'un film plastique de quelques microns d'épaisseur, qui enveloppe au moins partiellement ledit bourrelet 11. Ainsi, la membrane 13 présente une souplesse plus importante que celle du bourrelet de mousse 11.

Selon l'invention, la membrane souple 13 est rapportée (par exemple par collage), à une de ses extrémités 13E, sur la surface extérieure 2E de la coque creuse 2. Lorsque le masque 1A, 1 B est positionné sur le visage 9 du patient, la membrane souple 13 s'interpose entre le bourrelet 11 et le visage 9 sur lequel il prend appui.

En outre, la coque creuse 2 comporte une pluralité d'orifices de communication 14 ménagés en regard de la membrane 13, de manière à permettre le passage du gaz respiratoire 7 depuis la chambre interne 3 vers un espace intermédiaire E formé entre la membrane 13 et la surface extérieure 2E de la coque creuse 2. L'espace intermédiaire E se prolonge en outre entre la membrane 13 et au moins une partie de la surface S du bourrelet 11.

De préférence, les orifices de communication 14 sont régulièrement répartis autour de la coque creuse 2, de façon à assurer une répartition uniforme du gaz respiratoire 7 dans l'espace intermédiaire E.

Chacun des orifices 14 se présente avantageusement sous la forme d'un trou percé obliquement dans la paroi de la coque 2, de sorte que l'axe dudit orifice 14 soit incliné par rapport à la direction locale orthogonale à la portion de paroi de la coque 2, afin de faciliter le passage de gaz respiratoire 7 dans l'espace E.

Dans l'exemple de réalisation illustré sur les figures 1 à 3, l'autre extrémité 13I de la membrane 13 est libre et se prolonge vers l'intérieur de l'ouverture 8.

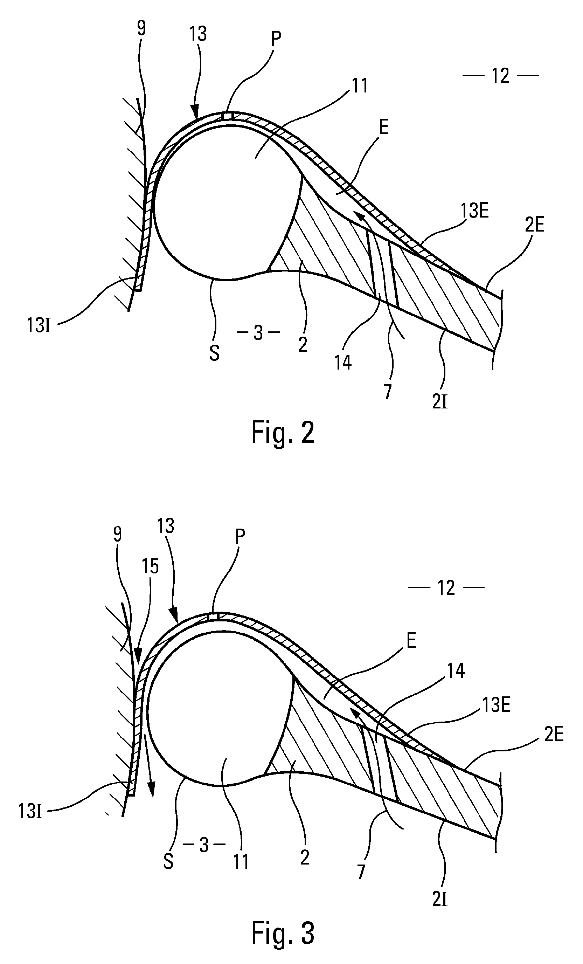

Lorsque le masque 1A est appliqué sur le visage 9 du patient, le bourrelet 11, à cause de sa rigidité relative et des irrégularités du relief du visage 9, ne peut s'appliquer uniformément sur ce dernier de façon rigoureusement étanche. Certes, sur la grande partie du contour du masque 1A, le bourrelet 11 s'applique avec étanchéité contre le visage 9, comme cela est illustré sur la figure 2, de sorte que la membrane 13 interposée est elle-même pressée entre le bourrelet 11 et le visage 9. En revanche, en certains endroits dudit bourrelet 11, celui-ci est maintenu écarté du visage 9 en laissant subsister un espace 15 avec ce dernier (voir la figure 3).

Dans ce dernier cas, une partie du gaz respiratoire 7, qui pénètre dans la coque rigide 2 par l'ouverture 4 (flèche 7) et passe dans la chambre interne 3, traverse les orifices de communication 14 pour circuler dans l'espace intermédiaire E en contournant le bourrelet 11 et aboutir de nouveau dans la chambre interne 3. La circulation du gaz 7 dans l'espace intermédiaire E conduit à la formation d'un coussin de gaz C entre au moins une portion de la surface S du bourrelet 11 et la membrane 13 en regard, tendue par la circulation de gaz dans l'espace E. Ce coussin de gaz C mou s'adapte aisément à la morphologie du patient, par simple contact de celui-ci sur la partie concernée du patient, ce qui assure une étanchéité uniforme entre le visage 9 et le masque 1A, suivant l'ouverture 8. Ainsi, la membrane 13 s'applique contre le visage 9 du patient en obturant l'espace 15.

En conséquence, la membrane souple 13 permet de parfaire l'étanchéité du masque d'assistance respiratoire 1A, aux emplacements où cette étanchéité ne peut être assurée par le bourrelet 11.

De plus, puisque la membrane 13 est souple et élastique et qu'elle présente une extrémité libre 13I dans l'ouverture 8, le masque respiratoire peut s'adapter automatiquement aux différentes morphologies des visages de dimensions différentes, la membrane souple 13 tendue se conformant avec ajustement sur ces visages.

Par ailleurs, lors de la circulation du gaz 7 dans l'espace intermédiaire E, l'extrémité libre 13I de la membrane 13 est plaquée contre le visage 9, ce qui optimise l'étanchéité en augmentant la surface de contact entre la membrane 13 et le visage 9. La zone annulaire ouverte formée entre l'extrémité libre 13I de la membrane 13 et le bourrelet 11 en regard définit un moyen d'échappement de gaz.

Il est à noter que, comme le montrent les figures 1 à 3, le masque 1A peut comporter une ou plusieurs perforations P, pratiquées dans la membrane 13 et destinées à permettre l'évacuation de gaz vicié expiré par le patient. En variante ou en complément, ces perforations pourraient être ménagées dans la coque creuse 2, entre l'extrémité 13E de la membrane 13 et l'entrée de gaz 4 (voir la figure 4).

Dans l'autre exemple de réalisation du masque 1B de l'invention représenté sur la figure 4, l'autre extrémité 131 de la membrane 13 est rapportée sur la surface intérieure 21 de la coque creuse 2 tournée vers la chambre 3. Elle est fixée, par exemple par collage, entre le bourrelet 11 et les orifices de communication 14, de sorte que ces derniers ne sont pas recouverts par la portion de la membrane 13 disposée à l'intérieur de la chambre 3.

Comme le montre la figure 4, plusieurs orifices d'échappement 16 sont ménagés dans la portion de la membrane 13 à l'intérieur de la chambre 3, de manière à permettre une évacuation, dans cette dernière, du gaz respiratoire 7 ayant circulé préalablement dans l'espace intermédiaire E.

Dans le cas où les orifices de communication 14 et d'échappement 16 sont en nombre identique, la section des orifices de communication 14 est avantageusement supérieure à celle des orifices d'échappement 16, de manière à assurer un gonflage permanent de la membrane souple 13.

Le processus selon lequel l'étanchéité est obtenue avec le masque 1 B de la figure 4 est semblable à celui décrit en regard du masque 1A des figures 1 à 3.

Dans la variante de réalisation illustrée schématiquement par la figure 5, l'appareil d'assistance respiratoire 17 conforme à la présente invention se présente sous la forme d'un dispositif d'intubation nasal comportant un élément tubulaire 18, dont le volume interne définit une chambre interne 19, qui est destiné à être introduit dans une narine 20 d'un patient. Du gaz respiratoire 7, provenant d'une source externe non représentée, est introduit, par une tubulure 6, dans l'élément tubulaire 18 à travers l'extrémité proximale 21 de ce dernier et est adressé aux poumons dudit patient à travers l'extrémité distale 22 dudit élément 18, débouchant dans la narine 20.

Le dispositif 17 comporte un organe d'appui 24 se présentant sous la forme d'un ballonnet de maintien et d'étanchéité, apte à venir s'appuyer contre la paroi interne 201 de la narine 20. Le ballonnet 24 est porté par la surface extérieure 18E de l'élément tubulaire 18 et est interposé entre ce dernier et la paroi interne 201.

Comme le montre la figure 5, la surface Si du ballonnet 24 est recouverte d'une membrane souple 25. Cette membrane 25 comporte une extrémité 25E rapportée (par exemple par collage) sur la surface extérieure 18E de l'élément tubulaire 18 et une extrémité libre interne 251 qui se prolonge vers l'ouverture formée par l'extrémité distale 22 de l'élément tubulaire 18.

En outre, l'élément tubulaire 18 comporte une pluralité d'orifices de communication 26 en regard de la membrane 25, pour que du gaz respiratoire 7 puisse être transféré depuis la chambre interne 19 vers un espace intermédiaire Ei. Cet espace Ei est formé entre la membrane 25 et la surface extérieure 18E de l'élément 18 et se prolonge entre la membrane 25 et la surface Si du ballonnet 24.

Les orifices 26, avantageusement répartis équi-angulairement sur une même section de l'élément tubulaire 18, sont percés de façon oblique par rapport à la direction locale orthogonale de la paroi de l'élément tubulaire 18.

Ainsi, du gaz respiratoire 7, provenant de la source, peut traverser les orifices 26 et circuler dans l'espace intermédiaire Ei, en contournant le ballonnet 24, ce qui gonfle la membrane 25 et forme un coussin de gaz Ci apte à s'adapter aux diverses irrégularités de la paroi interne 201 de la narine 20 du patient.

On comprendra aisément que, de façon semblable à ce qui a été expliqué ci-dessus en regard des figures 1 à 4, la membrane 25 est apte à assurer l'étanchéité entre la paroi interne 201 de la narine 20 et la sortie 22 de l'élément tubulaire 18, aux endroits où le ballonnet 24 présente des défauts d'étanchéité. La zone annulaire ouverte formée entre l'extrémité libre 251 de la membrane 25 et l'élément tubulaire 18 définit un moyen d'échappement de gaz.

Par ailleurs, une ou plusieurs perforations Pi peuvent être ménagées dans la paroi de l'élément tubulaire 18, entre l'extrémité 25E de la membrane 25 et l'extrémité proximale 21, afin de réaliser une évacuation du gaz vicié rejeté par le patient et circulant dans la chambre 19.

## Revendications

1. Appareil d'assistance respiratoire permettant d'amener, dans les poumons d'un patient, du gaz respiratoire (7) provenant d'une source extérieure, ledit appareil (1 A, 1 B, 17) comportant :
- un corps creux (2, 18), dont le volume interne définit une chambre interne (3, 19) pourvue d'une entrée (4, 21) de gaz respiratoire destinée à être reliée à ladite source et d'une sortie de gaz respiratoire destinée à être reliée à une voie respiratoire dudit patient ;
- une ouverture (8, 22) qui constitue ladite sortie de gaz respiratoire ;
- un organe d'appui (11, 24) qui borde le voisinage du contour de ladite ouverture (8, 22) et qui est destiné à prendre appui sur une partie (9, 201) dudit patient pour assurer l'étanchéité entre ladite sortie de gaz respiratoire et l'extérieur (12) ; et
- une membrane (13, 25) souple qui enveloppe, au moins partiellement, ledit organe d'appui (11, 24),
**caractérisé :**
- **en ce que** ladite membrane souple (13, 25) est rapportée, à une de ses extrémités (13E, 25E), sur la surface (2E, 18E) dudit corps creux (2, 18) orientée vers l'extérieur ;
- **en ce que** ladite membrane souple (13, 25) est apte à s'interposer entre ledit organe d'appui (11, 24) et la partie (9, 201) dudit patient sur laquelle il prend appui ; et
- **en ce que** ledit corps creux (2, 18) comporte au moins un orifice de communication (14, 26) ménagé en regard de ladite membrane (13, 25),
de sorte que du gaz respiratoire est apte à traverser ledit orifice de communication (14, 26), à circuler, depuis ladite chambre (3, 19), dans un espace intermédiaire (E, Ei) formé entre ladite membrane (13, 25) et la surface extérieure (2E, 18E) dudit corps creux (2, 18), et à contourner l'organe d'appui pour retourner dans ladite chambre interne (3, 19) à proximité de ladite ouverture (8, 22).

2. Appareil selon la revendication 1,
**caractérisé en ce que** ledit corps creux (2, 18) comporte une pluralité d'orifices de communication (14, 26) régulièrement répartis sur ce dernier.

3. Appareil selon la revendication 1 ou 2,
**caractérisé en ce que** ledit orifice de communication (14, 26) se présente sous la forme d'un trou percé de façon oblique dans la paroi dudit corps creux (2, 18), de sorte que l'axe dudit orifice (14, 26) soit incliné par rapport à la direction locale orthogonale à ladite paroi.

4. Appareil selon l'une des revendications 1 à 3,
**caractérisé en ce que** ladite membrane souple (13, 25) est réalisée dans un film plastique de quelques micromètres d'épaisseur.

5. Appareil selon l'une des revendications 1 à 4,
**caractérisé en ce qu'**il se présente sous la forme d'un masque (1 A, 1 B), dans lequel ledit corps (2) est une coque creuse destinée à être appliquée, par ladite ouverture (8), sur le visage (9) d'un patient en enfermant au moins le nez (10) de celui-ci, l'entrée (4) du gaz respiratoire étant prévue dans le fond de ladite coque (2) et ledit organe d'appui (11) présentant la forme d'un bourrelet suivant le contour de l'ouverture de ladite coque et interposé entre ladite ouverture (8) et le visage(9) dudit patient.

6. Appareil selon la revendication 5,
**caractérisé en ce que** l'autre extrémité (131) de ladite membrane (13) est libre et se prolonge vers l'intérieur de ladite ouverture (8).

7. Appareil selon la revendication 5,
**caractérisé :**
- **en ce que** l'autre extrémité (131) de ladite membrane (13) est rapportée sur la surface (21) de ladite coque creuse (2) tournée vers ladite chambre (3), entre ledit bourrelet (11) et ledit orifice de communication (14) ;
- et **en ce qu'**au moins un orifice d'échappement (16) est ménagé dans la portion de ladite membrane (13) agencée à l'intérieur de ladite chambre (3), de manière à permettre une évacuation du gaz respiratoire (7) dans cette dernière.

8. Appareil selon la revendication 7,
**caractérisé en ce que** la section dudit orifice de communication (14) est supérieure à celle dudit orifice d'échappement (16).

9. Appareil selon l'une des revendications 1 à 4,
**caractérisé en ce qu'**il se présente sous la forme d'un dispositif d'intubation nasal (17), dans lequel ledit corps creux (18) est un élément tubulaire destiné à être introduit dans une narine (20) du patient, lesdites entrée (21) et sortie (22) de gaz respiratoire étant formées par les extrémités opposées dudit élément tubulaire (18) et ledit organe d'appui (24) se présentant sous la forme d'un ballonnet porté par la surface extérieure dudit élément tubulaire (18) et interposé entre celui-ci et la paroi interne (201) de ladite narine (20).

10. Appareil selon la revendication 9,
**caractérisé en ce que** ladite membrane (25) recouvre intégralement ledit organe d'appui (24) et se prolonge, par son autre extrémité libre (251), vers l'intérieur de ladite ouverture (22).
